# EUROPEAN PATENT APPLICATION

(11) **EP 4 382 066 A1**
(43) Date of publication of application: **12.06.2024**
(21) Application number: 22212139.4
(22) Date of filing: 08.12.2022
(51) Int. Cl.: A61B 34/20

(54) **LCQ POSITION MARKERS**

(71) Applicant: Koninklijke Philips N.V., 5656 AG Eindhoven (NL)
(72) Inventor: GLEICH, Bernhard, Eindhoven (NL); RAHMER, Juergen Erwin, Eindhoven (NL); SCHMALE, Ingo, 5656AG Eindhoven (NL)
(74) Representative: Philips Intellectual Property & Standards

(57) **Abstract**

The present disclosure relates to device tracking. Described is an improved passive marker device (10). The passive marker device (10) comprises a coil element (12), a mechanical resonator (14), and a circuit element (16). The coil element is coupled to the mechanical resonator for producing a response signal having an operating frequency corresponding a mechanical resonating frequency of the mechanical resonator and a signal decay having a decay time constant in response to an excitation pulse, wherein the excitation pulse is a magnetic or electromagnetic excitation pulse. The circuit element is configured to limit a power transferred to the mechanical resonator when the at least one excitation pulse has an excitation amplitude equal to or greater than a threshold value.

## Description

### FIELD OF THE INVENTION

The present invention relates to passive marker device(s) to be tracked, passive marker device arrangement(s), a tracking system(s), and methods related thereto.

### BACKGROUND OF THE INVENTION

Device tracking is useful e.g., in certain medical procedure. For example, a marker device may be attached to a medical device, such as a medical interventional device, during the procedure. A system for miniature markers and sensors has been recently described in WO2019243098, which is based on the usage of so-called micro-magnetic oscillators (MMOs). In these systems, the initiation of mechanical oscillations in the MMOs in response to a magnetic or electromagnetic excitation filed is used for tracking the marker devices comprising these MMOs, and therefore, the devices these marker devices are attached to.

However, for MMOs, the signal-to-noise ratio (SNR) scales with the square of the linear dimensions of the MMO devices. Accordingly, these MMO devices become less and less beneficial with increasing spaces available.

### SUMMARY OF THE INVENTION

There is a need to provide an improved passive tracker device.

The object of the present invention is solved by the subject-matter of the independent claims, wherein further embodiments are incorporated in the dependent claims. It should be noted that the following described aspects of the invention apply also for the passive marker device to be tracked, the passive marker device arrangement, and the tracking system.

According to a first aspect of the present invention, there is provided a passive marker device. The passive marker device comprises a coil element, a mechanical resonator, and a circuit element. The coil element is coupled to the mechanical resonator for producing a response signal having an operating frequency corresponding a mechanical resonating frequency of the mechanical resonator and a signal decay having a decay time constant in response to an excitation pulse. The excitation pulse is a magnetic or electromagnetic excitation pulse. The circuit element is configured to reduce a power transferred to the mechanical resonator when the at least one excitation pulse has an excitation amplitude equal to or greater than a threshold value.

One of the challenges to be solved may be that passive marker devices near the transmit coil system may be subject to much stronger excitation. This may lead to a too large voltage across the quartz that might destroy it, and/or a situation in receive mode, where the signals received from the nearby passive marker devices are far stronger than those from the distant ones, and hence make those weaker signals difficult to detect.

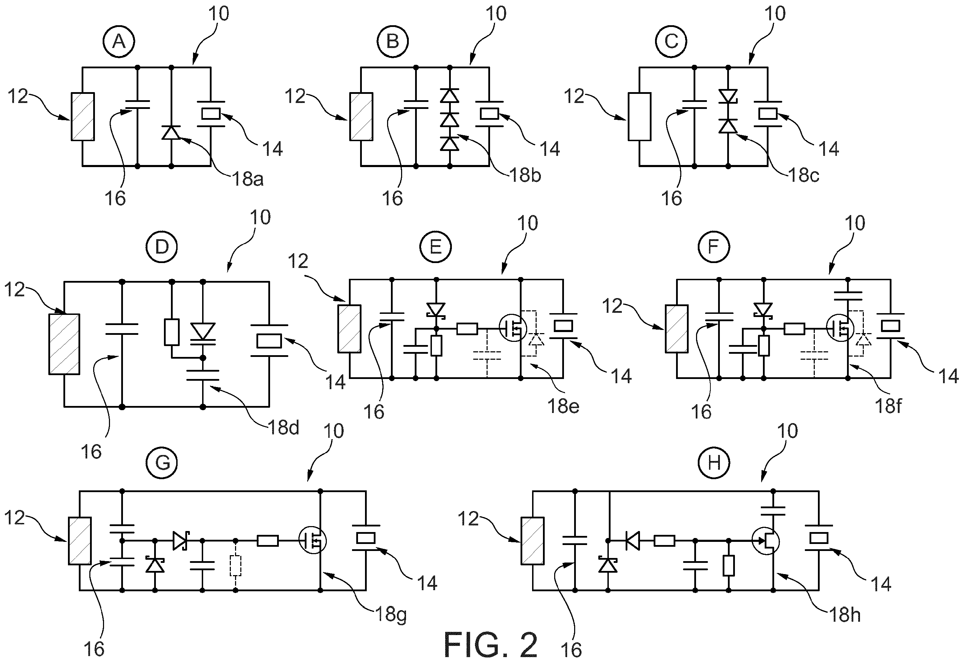

To that end, the present disclosure provides a passive marker device with a circuit element to limit or reduce the power transferred to the mechanical resonator at a high excitation amplitude, i.e., when the passive marker device is near a coil system. The passive marker device will be described in detail hereinafter and in particular with respect to the examples shown in FIGS. 2A-2H.

In more sophisticated schemes, the coupling between the coil element and the mechanical resonator may be reduced or even eliminated for a duration much longer than the oscillation period. This will be described in detail hereinafter and in particular with respect to the examples shown in FIGS. 2D-2H.

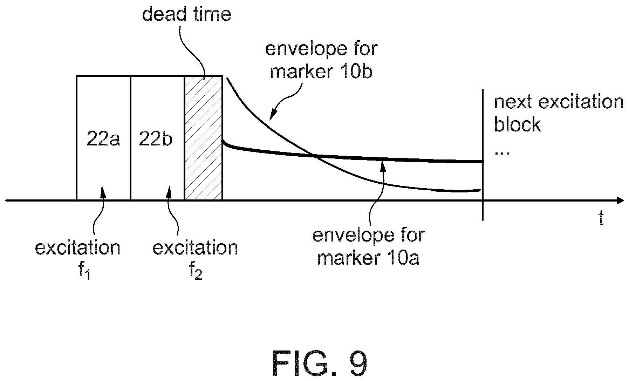

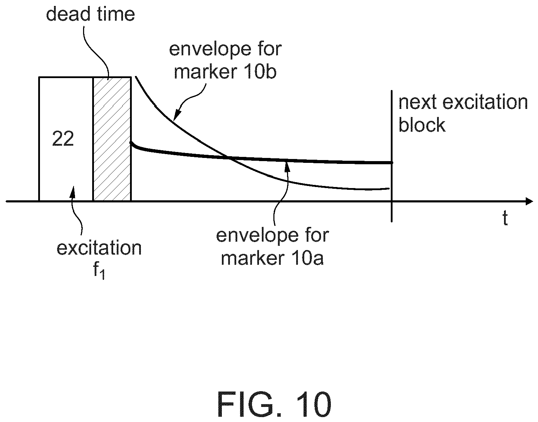

This may furthermore be combined with phase-altering excitation pulses to get the desired low amplitude of the close passive marker devices while the amplitude of the more distant passive marker devices is not affected significantly. Assuming a simple circuit where the amplitude is capped, the sequence during the send phase (i.e., in the excitation time slot) has two phases: first a low amplitude signal is sent, then a higher amplitude signal having about the same duration, but with opposite phase is sent. This has the effect, that at the near position, due to capping of the signal, a near zero excitation is achieved. At the position far away from the coil array, the relative amplitudes of the two 180° phase-shifted signal do not cancel and hence a relatively high signal amplitude is achieved. This is because at this far-away position the excitation is linear with the applied field and the weak field from the first phase does not cancel the strong field from the second phase, which will be described in detail hereinafter and in particular with respect to the exemplary excitation and recording schemes shown in FIGS. 9 and 10.

According to an embodiment of the present invention, the circuit element comprises at least one of:
- a diode configured to limit a voltage across the mechanical resonator;
- a plurality of diodes connected in series configured to limit a voltage of the mechanical resonator; or
- a pin diode configured to limit a voltage across the mechanical resonator.
This will be explained in detail hereinafter and in particular with respect to the examples shown in FIGS. 2A-2C.

According to an embodiment of the present invention, the circuit element is configured to reduce a coupling between the coil element and the mechanical resonator.

*In other words, the coupling between the coil element and the mechanical resonator can be reduced or even eliminated for a duration much longer than the oscillation period.*

The reason to change the coupling between coil and quartz was twofold: first not to destroy the quartz and second not to saturate the receive amplifier (or have excessive spectral leakage). For the latter reason, a suitable sequence has to be provided. Assuming a simple circuit where the amplitude is capped, the sequence during the send phase (i.e., in the excitation time slot) has two phases: first a low amplitude signal is sent, then a higher amplitude signal having about the same duration, but with opposite phase is sent. This has the effect, that at the near position, due to capping of the signal, a near zero excitation is achieved. At the position far away from the coil array, the relative amplitudes of the two 180° phase-shifted signal do not cancel and hence a relatively high signal amplitude is achieved. This will be described in detail hereinafter and in particular with respect to the exemplary excitation and recording schemes shown in FIGS. 9 and 10.

According to an embodiment of the present invention, the circuit element comprises a varactor diode and a resistor parallel to the varactor diode.
This will be described in detail hereinafter and in particular with respect to the example shown in Fig. 2D. According to an embodiment of the present invention, the circuit element further comprises one of:
- a field effect transistor configured to clamp a charging action;
- a field effect transistor configured to clamp a charging action and a series capacitor connected to the field effect transistor;
- a field effect transistor configured to clamp a charging action and a series capacitor connected to the field effect transistor, wherein the field effect transistor is connected to a capacitive voltage divider of the capacitive element; or
- a normally-on field effect transistor as a switching element.

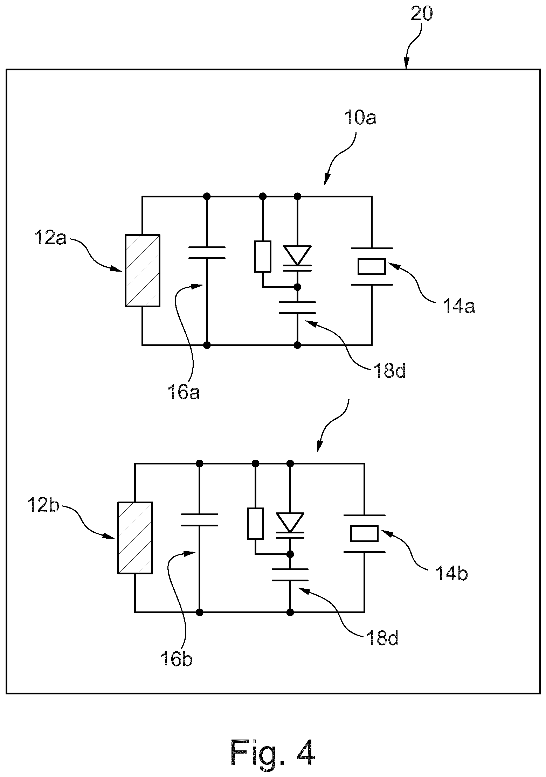

This will be described in detail hereinafter and in particular with respect to the example shown in Figs. 4E-4H.

According to a second aspect of the present invention, there is provided a passive marker arrangement. The passive marker arrangement comprises a first passive marker device and a second passive marker device. Each of the first and second passive marker device comprises a coil element and a mechanical resonator, wherein the coil element is coupled to the mechanical resonator for producing a response signal having an operating frequency corresponding a mechanical resonating frequency of the mechanical resonator and a signal decay having a decay time constant in response to an excitation pulse. The excitation pulse is a magnetic or electromagnetic excitation pulse. The first passive marker device and the second passive marker device are mounted on a mechanical arrangement at an angle with respect to each other, such that the coil element of the first passive marker device is non-parallel to the coil element of the second passive marker device.

In some examples, it may be preferred to track six (6) mechanical degrees of freedom (DOF) of movement of a device, e.g., a medical device, in three-dimensional space. However, the efficiency of a single coil is proportional to the sine of the angle between the local excitation field and the coil plane. Thus, if the coil element of the passive marker device is parallel to the local excitation field, the coil element will not be excited and thus no oscillation response can be generated by the passive marker device. One solution is to switch to a different direction of the field to excite the coil element. However, such approach may be slow and inefficient. For example, if an application has 40-100 Hz acquisition rate and the passive marker device works at a few kHz, long and complex excitation sequences may be required. Therefore, with a single coil it may be difficult to obtain the desired time resolution for a medical application. Additionally, if the angle between coil and field is low, the response signal is low as well, which may result in lower SNR.

To that end, according to this embodiment, the orientation of two passive marker devices may be physically linked via a mechanical arrangement, e.g., a housing. The two passive marker devices are arranged in a non-parallel to each other. Thus, if one coil element of the passive marker device arrangement is parallel to the local excitation field, the other coil element of the passive marker device arrangement is non-parallel (e.g., orthogonal or almost orthogonal) to the local excitation field and can generate a higher response signal to achieve a higher SNR.

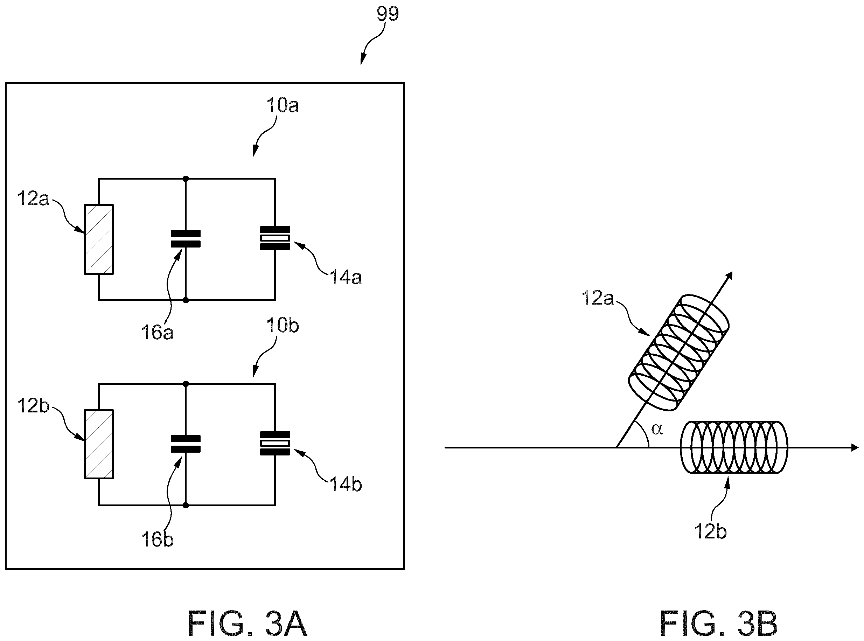

This will be described in detail hereinafter and in particular with respect to the example shown in Figs. 3A, 3B, and 4.
According to an embodiment of the present invention, at least one of the first passive marker device and the second passive marker device is a passive marker device according to the first aspect and any associated example.

This will be described in detail hereinafter and in particular with respect to the example shown in Fig. 4.

According to an embodiment of the present invention, the angle is in a range from about 45° to about 135°, optionally in a range from about 85° to about 95°, preferably about 90°.

According to an embodiment of the present invention, the first passive marker device and the second passive marker device are configured to have operating frequencies with a frequency spacing less than a threshold value and to have different decay time constants. Alternatively, the first passive marker device and the second passive marker device are configured to have operating frequencies with a frequency spacing equal to or greater than a threshold value.

In other words, it is possible to use decay time space and/or the frequency space to distinguish the first passive marker device and the second device. In some examples, both passive marker devices may have same or similar frequencies, but with different decay time constants. Alternatively, both marker devices may have different frequencies.

According to a third aspect of the present invention, there is provided a tracking system. The tracking system comprises a plurality of passive marker devices, an excitation filed generator, and a tracking device. The plurality of passive marker devices comprises a passive marker device according to the first aspect and any associated example and/or a passive marker arrangement according to the second aspect and any associated example. The excitation field generator is configured to generate at least one excitation pulse to excite at least two passive marker devices in simultaneous manner or in a sequential manner within one excitation time slot. The tracking device is configured to detect the response signals generated by the at least two passive marker devices and to differentiate the at least two passive marker devices on the basis of the operating frequencies of the response signals and the decay time constants of the response signals.

For most clinical applications, more than one devices must be tracked. This is especially the case, if a 6 DOF sensor is necessary as this alone needs two passive marker devices. The classical way to address different passive marker devices is to use frequency selective excitation pulses and to design (i.e., tune) and operate all markers at different frequencies. However, the limitation is that for a desired fast repetition time, the excitation pulses need to be short and significant spectral overlap may occur. When it comes to LC resonator with quartz resonators, an additional limitation is that only a limited number of quartz frequencies are available from components-of-the-shelf. While manufacturers can tune their crystals to any desired frequencies, the relatively low market volume makes a special design less economically viable.

In order to speed up the detection, the tracking system as disclosed herein can excite two or more marker devices in one excitation time slot and use decay time space in addition to the frequency space to distinguish the passive marker devices. Exemplary excitation and recording schemes are described in detail hereinafter and in particular with respect to the examples shown in Figs. 9 and 10. According to an embodiment of the present invention, the tracking device is configured to detect the response signals generated by the two or more passive marker devices in one or more reception time slots according to the decay time constants of the response signals.

In some examples, as described with respect to the exemplary excitation and recording schemes shown in Figs. 9 and 10, the response signals may be detected in one reception time slot.

In some other examples, for a high number of passive marker devices, the excitation phase may be so long that some of the signal has decayed too much before the reception phase (i.e., the reception time slot). In such cases, the response signals generated by the two or more passive marker devices may be detected in a plurality of reception time slots according to the decay time constants of the response signals.

According to an embodiment of the present invention, the plurality of passive marker devices comprises two or more passive marker devices configured to have operating frequencies with a frequency spacing less than a threshold value and to have different decay time constants.

For example, two or more passive marker devices may have essentially the same operating frequency, e.g., the operating frequencies are less than a defined threshold value. It is for example possible to excite two passive marker devices simultaneously, if the coil current pattern is distinct enough. This will be described in detail hereinafter and in particular with respect to the exemplary excitation and recording scheme shown in Fig. 10.

According to an embodiment of the present invention, the plurality of passive marker devices comprises two or more passive marker devices configured to have operating frequencies with a frequency spacing equal to or greater than a threshold value. The excitation field generator is configured to generate a sequence of frequency selective excitation pulses within the excitation time slot to excite at least two of the two or more passive marker devices in a sequential manner.

*For example, it is possible to use frequency selective excitation pulses to excite two or more passive marker devices at different frequencies.* This will be described in detail hereinafter and in particular with respect to the exemplary excitation and recording scheme shown in Fig. 9.

According to an embodiment of the present invention, the excitation field generator is configured to sort the frequency selective excitation pulses according to the decay time constants of the at least two passive marker devices to be excited.

This will be described in detail hereinafter and in particular with respect to the exemplary excitation and recording scheme shown in Fig. 9.

According to an embodiment of the present invention, the excitation field generator is configured to generate a sequence of phase-altering excitation pulses to excite at least two passive marker devices having different distances to the excitation field generator. The sequence of phase-altering excitation pulses comprises a first signal with a low amplitude and a second signal having a higher amplitude, wherein the first signal and the second signal have opposite phases.

*For examples, phase-altering excitation pulses may be used to get the desired low amplitude of the close passive marker device(s) while the amplitude of the more distant passive marker device(s) is not affected significantly.*

### BRIEF DESCRIPTION OF THE DRAWINGS

These and other aspects of the invention will be apparent from and elucidated further with reference to the embodiments described by way of examples in the following description and with reference to the accompanying drawings, in which
Fig. 1 schematically shows an example of a passive marker device.
Figs. 2A-2H schematically show some examples of the circuit element.
Figs. 3A-3B shows an exemplary passive marker arrangement.
Fig. 4 shows a further exemplary passive marker arrangement.
Figs. 5A-5E show exemplary circuits to change the time constant of the signal decay after excitation.
Fig. 6 schematically shows an example of a tracking system.
Fig. 7 schematically shows a further example of a tracking system.
Fig. 8 illustrates a general excitation and recording scheme.
Fig. 9 illustrates the basic principle of an exemplary improved excitation scheme.
Fig. 10 illustrates the basic principle of a further exemplary improved excitation scheme.
Fig. 11 illustrates a flowchart describing a method for tracking a plurality of marker device.

It should be noted that the figures are purely diagrammatic and not drawn to scale. In the figures, elements which correspond to elements already described may have the same reference numerals. Examples, embodiments or optional features, whether indicated as non-limiting or not, are not to be understood as limiting the invention as claimed.

### DETAILED DESCRIPTION OF EMBODIMENTS

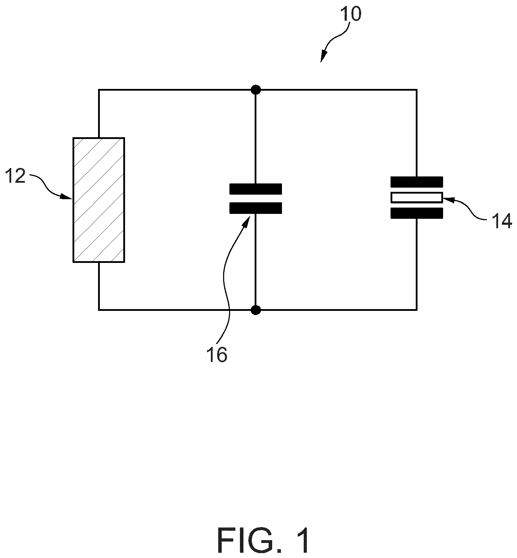

Fig. 1 schematically shows an example of a passive marker device 10 (e.g., a passive marker) that is attachable to a device, such as a medical device, for being tracked by a tracking system. The passive marker device 10 comprises a coil element 12 such as a coil and a mechanical resonator 14.

The coil element 12 may refer to an element comprising and/or corresponding to a magnetic coil arrangement having a particular number of windings. The coil element 12 may be an off-the-shelf magnetic coil having an appropriate number of windings, an appropriate size, and an appropriated distance between the windings. The amount, size, and distance between the windings may be particular determined based on the desired magnetic properties of the coil element. The coil element 12 may comprise or be made of a copper. The coil element 12 may, alternatively or additionally, comprise or to be made of silver. In some examples where the passive marker device is supposed to be transparent to radiation, the coil element 12 may comprise or be made of aluminum. In some examples where the passive marker device is supposed to be opaque to radiation, the coil element may comprise or be made of gold.

The mechanical resonator 14 is an element that is connected to the coil element 12 in order to respond to the coil element's voltage output in response to an externally applied magnetic or electromagnetic field by respectively deforming and, thus, starting to perform mechanical oscillations. The mechanical resonator 14 may comprise or be made of a crystalline material. The crystalline material may be chosen such as to have sufficient piezoelectric properties. The crystalline material may comprise and/or correspond to a quartz crystal. Quartz is a material for piezoelectric resonator, and it is resonance frequency is known. Accordingly, it is possible to provide a passive marker device that comprises the coil element and the mechanical resonator in which the components may be selected such as to provide an electrical field having a frequency that is close to or corresponds to the mechanical resonance frequency of the quartz crystal. This may allow to obtain a high quality factor for the passive marker device. A further benefit of using quartz resides in the fact that quartz may be used in serial or parallel resonance as both eigen frequencies are very close to each other. In addition, the resonance frequency may be slightly adjusted by respectively providing additional elements, e.g., a trimmer in parallel or in series to the quartz crystal, depending on whether the quartz crystal is connected in parallel or in series. This may allow to compensate for manufacturing tolerances or the like. Further crystalline materials with similar properties may likewise be foreseen. The mechanical resonator 14 may comprise a main body and at least one prong attached thereto. For example, the resonator element may be of a fork type, such as a tuning fork type, having two prongs attached to the main body. The mechanical resonator 14 with the shape of a tuning fork may improve the resonating properties and may also be beneficial in terms of using the passive marker device for sensing physical properties.

The coil element 12 may be arranged at a distance from the mechanical resonator 14. The coil element 12 and the mechanical resonator 14 may, for this purpose, be connected via a respective connection portion. In some examples, where space has to be saved, the distance may be saved by providing the windings around the mechanical resonator such that there is a space between the wingdings of the coil element 12 and the mechanical resonator 14. The dimensions of this space may be chosen appropriately according to the dimensioning of the passive marker device 10. In some examples, the arrangement between the coil element 12 and the mechanical resonator 14 may be such that the mechanical resonator 14 is provided in the coil element and extends along its axis.

When an externally magnetic or electromagnetic field is applied, the externally applied magnetic or electromagnetic filed may act on the coil element 12. In response to this, the coil element 12 may transduce the externally applied magnetic or electromagnetic field in to a respective output voltage. The coil element 12 is electrically connected to the mechanical resonator 14 to feed the output voltage to the input/output terminals of the mechanical resonator 14. The mechanical resonator 14, having piezoelectric properties, is then deformed by the voltage applied from the coil element 12. Accordingly, the mechanical resonator start performing mechanical oscillations. Hereby, the deformation is dependent on the frequency component of the applied output voltage provided by the coil element 12, which, in turn, is dependent on the frequency components of the externally applied magnetic or electromagnetic excitation field. As stated, if the frequency components are provided to be a mechanical resonance frequency of the mechanical resonator or within a range around the mechanical resonance frequency of the mechanical resonator, the mechanical oscillations are excited in the resonant mode. That is, the mechanical resonator 14 oscillates close to its mechanical resonance frequency. The respective oscillations may the persist for some time, independent whether or not voltage is provided from the coil element 12, thereby causing a signal decay having a decay time constant. The deforming of the mechanical resonator 12 then causes a piezoelectric voltage to be generated and output to the coil element 12. This causes a current through the coil element. In response to this, the coil element 12 produces a magnetic field which may then be detected as an oscillation response by the tracking system.

In some examples, as shown in Fig. 1, the passive marker device 10 may further be provided with a capacitive element 16 such as a capacitor. The capacitive element 166 may be connected in parallel to the coil element 12 such as a coil. This essentially provides a resonator circuit which has an LC resonator and a mechanical resonator combined with each other. The capacitive element 16 may be selected to have a capacitance that amplifies the output voltage provided by the coil element 12 in response to the externally applied magnetic or electromagnetic excitation filed. The amplified output voltage may then be provided to the mechanical resonator 16 to cause stronger deformations therein, and hence a higher level of oscillation in the mechanical resonator. This, in turn, may result in a higher piezoelectric voltage acting upon the coil element 12 to improve the signal strength. In some examples, the value of the capacitance of the capacitive element may be chosen such that the resonance frequency of the coil element in the LC resonator is equal to or close to the mechanical resonance frequency of the mechanical resonator 16, such as quartz crystal. This may allow to reduce the amount of windings needed, as the loss during oscillator of the resonator circuit becomes low. With less windings, the manufacture of the passive marker device may also be simplified. Passive marker devices that comprise the coil element 12, the mechanical resonator 14 in form of a quartz crystal, and the capacitive element 16 may also be referred to as "LCQ" markers. Compared to micro-magnetic oscillators-based markers, LCQ markers may offer a larger operation distance from the coil array, and have a better SNR. In the following, the exemplary passive marker devices 10 are illustrated in the form of LCQ markers by way of example, but it will be appreciated that the passive marker devices 10 may be other types of passive marker devices.

One problem associated with the passive marker devices may be the possibility of too high signal amplitude of the passive marker devices. This may be due to the operation of two or more passive marker devices at the same frequency and one being much closer to the coil array than the others. This may also happen if they are not tuned to the same frequency but subject to spectral leaking. This problem may be overcome by utilizing a circuit element to limit or reduce the power transferred to the crystal at a high excitation amplitude, i.e., when near the coil system. The circuit element may be a non-linear circuit element.

Figs. 2A to 2H schematically show some examples of the circuit element 18. In particular, Figs. 2A-2C show some exemplary circuit elements configured to limit the voltage across the mechanical resonator, while Figs. 2D-2E show some exemplary circuit elements configured to reduce or even eliminate the coupling between the coil element and the mechanical resonator for a duration much longer than the oscillation period.

Fig. 2A shows an exemplary passive maker device 10 with an exemplary circuit element 18a. In this example, the exemplary circuit element 18a is a diode configured to limit the voltage across the mechanical resonator. With the diode, only one direction may be needed as this may already reduce the energy stored in the tank circuit sufficiently. The advantage of this exemplary circuit element may be that it is simple and cost effective. The use of PIN-diodes may allow for a relatively low additional capacitance so that the effects before clamping may be relatively minor.

Fig. 2B shows an exemplary passive maker device 10 with a circuit element 18b to increase the voltage at which the voltage limiting effect occurs. In this example, the circuit element 18b comprises a plurality of diodes and each diode in series increases the voltage by roughly 0.5V.

Fig. 2C shows an exemplary passive maker device 10 with an exemplary circuit element 18c. In this example, the exemplary circuit element 18c comprises only one (PIN-)diode 18c. The Z-diode sets may largely the limiting voltage by its breakdown, while avoiding the drawback of potential high number of needed diodes.

Fig. 2D shows an exemplary passive maker device 10 with an exemplary circuit element 18d configured to limit the voltage at the mechanical resonator by detuning the tank circuit. In this example, the exemplary circuit element 18d comprises a varactor diode and a resistor parallel to the varactor diode. In response to the applied magnetic or electromagnetic field, the varactor diode starts to detune the tank circuit when it becomes conductive and charges the series capacitor and itself. The detuning action may be not limited to the immediate voltage but persist potentially for a long time. The persistence time may be set to the right duration, i.e., to about the dead time. A resistor parallel may be provided and arranged in parallel to the varactor diode. An additional advantage of this circuit is as follow: during the dead time, the tank circuit is detuned and hence the energy stored persists in the quartz resonator increasing the signal during the receive time.

Fig. 2E shows an exemplary passive marker device 10 with an exemplary circuit element 18e. In this example, the exemplary circuit element 18e comprises a field effect transistor (FET) to clamp the charging action. This exemplary circuit may also allow to prolong the switching pattern of the excitation sequence. Thus, after reaching a critical voltage, the circuit is muted for a prolonged time. In this circuit, there are two elements to store charge to perform the delayed action. First, there is the capacitor in series to the Schottky diode. This charge is removed by the resistor parallel to this capacitor, forming a resistor-capacitor (RC) circuit. The second RC circuit is formed by the gate resistor and the gate capacitance (depicted as dotted capacitor). Depending on the excitation sequence, only one RC circuit for delayed action may be necessary, and, in this case, the gate resistor and the capacitor in series to the diode may be omitted. The diode generates a direct current (DC) voltage and at some point, the voltage may be high enough to switch the transistor. This then shorts the tank circuit. The practical implementations of field effect transistors, usually a metal oxide semiconductor field effect transistor (MOSFET), have a body diode (dotted diode) which may limit the tank circuit voltage to 0.5V peak.

Fig. 2F shows an exemplary passive marker device 10 with an exemplary circuit element 18f, which may avoid the problem by adding a series capacitor to the transistor. The diode's rectification results in a voltage at the transistors drain eventually stopping the DC current flow. In the transistors "ON" state, i.e., when a suitably high gate voltage is reached, the transistor series capacitor effectively becomes a parallel capacitor to the tank circuit detuning it. The voltage of action can be determined by the selected transistor (threshold voltage), or by a circuit as depicted in Fig. 2G.

Fig. 2G shows an exemplary passive marker device 10 with an exemplary circuit element 18g. In this example, the tank circuit capacitance is replaced by a capacitive voltage divider. A charge pump (e.g., two diodes) provides the desired gate voltage. A remaining issue with the transistors is their voltage-dependent output capacitance. This is largely avoided in circuit shown in Fig. 2H.

Fig. 2H shows an exemplary passive marker device 10 with an exemplary circuit element 18h. In this example, a normally-on field effect transistor (in this case an n-channel junction field effect transistor (jFET)) is used as switching element. Until the gate is negative enough to make the transistor non-conductive, the series capacitor is in parallel to the rest of the tank circuit capacitance. The diode for generating the negative voltage has a series resistor typically in the megaohm range. Therefore, the diodes non-linear action hardly changes the resonance in the circuit. The parallel RC circuit between gate and source governs the discharge time constant and hence the duration of the detuning. This circuit also allows for a prolonged muting duration by adding power at the higher frequency after the jFET switched off.

The passive marker device shown in Figs. 1 and 2A-2H may be used to track 6 DOF of movement of a device, e.g., a medical device, in three-dimensional space. However, the efficiency of a single coil is proportional to the sine of the angle between the local excitation field and the coil plane. Thus, if the coil element of the passive marker device is parallel to the local excitation field, the coil element will not be excited and thus no oscillation response can be generated by the passive marker device. One solution is to switch to a different direction of the field to excite the coil element. However, such approach may be slow and inefficient. For example, if an application has 40-100 Hz acquisition rate and the passive marker device works at a few kHz, long and complex excitation sequences may be required. Therefore, with a single coil it may be difficult to obtain the desired time resolution for a medical application. Additionally, if the angle between coil and field is low, the response signal is low as well, which may result in lower SNR.

Fig. 3A shows an exemplary passive marker arrangement 20 to address one or more of the above-mentioned problems. The passive marker arrangement 20 comprises a first passive marker device 10a and a second passive marker device 10b.

The first passive marker device 10a comprises a first coil element 12a, a first mechanical resonator 14a, and a first capacitive element 16a. The first coil element 12a is coupled to the first mechanical resonator 14a for producing a response signal having an operating frequency corresponding a mechanical resonating frequency of the mechanical resonator and a signal decay having a decay time constant in response to an excitation pulse.

The second passive marker device 10b comprises a second coil element 12b, a second mechanical resonator 14b, and a second capacitive element 16b. The second coil element 12b is coupled to the second mechanical resonator 14b for producing a response signal having an operating frequency corresponding a mechanical resonating frequency of the mechanical resonator and a signal decay having a decay time constant in response to an excitation pulse.

The first passive marker device 10a and the second passive marker device 10b are mounted on a mechanical arrangement (not shown) at an angle with respect to each other, such that the first coil element 12a of the first passive marker device 10a is non-parallel to the second coil element 12b of the second passive marker device 10b.

Fig. 3B illustrates an exemplary arrangement of the first coil element 12a of the first passive marker device 10a with respect to the second coil element 12b of the second passive marker device 10b. As shown in Fig. 3B, the first coil element 12a of the first passive marker device 10a and the second coil element 12b of the second passive marker device 10b are arranged at an angle *α* relative to each other. As the first coil element 12a of the first passive marker device 10a is non-parallel to the second coil element 12b of the first passive marker device 10a, the angle α is not equal to 0° or 180°. For example, the angle α is greater than 0° and less than 180°. For example, the angle α may be in a range from about 45° to about 135°. Preferably, the angle α is in a range from about 85° to about 95°, and more preferably about 90°.

As the first coil element 12a of the first passive marker device 10a and the second coil element 12b of the second passive marker device 10b are arranged in a non-parallel manner, if one coil element, e.g., the first coil element 12a, is parallel to the local excitation field, the other coil element, e.g., the second coil element 12b, is non-parallel to the local excitation field. Thus, at least one coil element in the passive marker arrangement 20 will be excited to provide an oscillation response to be tracked by the tracking system. In this way, it is not required to switch the direction of the excitation field to excite the coil element, thereby achieving a fast and efficient acquisition. Preferably, the first coil element 12a of the first passive marker device 10a and the second coil element 12b of the second passive marker device 10b are arranged orthogonal (about 90°) or almost orthogonal (e.g., in a range from about 85° to about 95°) with respect to each other in order to generate a higher response signal. For example, if one coil element of the passive marker device arrangement is parallel to the local excitation field, the other coil element of the passive marker device arrangement is perpendicular to the local excitation field and can generate a higher response signal to achieve a higher SNR.

In some examples, one or both of the first passive marker device 10a and the second passive marker device 10b may comprise a circuit element to limit a power when the local excitation field has an excitation amplitude equal to or greater than a threshold value.

Fig. 4 shows a further example of a passive marker device arrangement 20, in which both the first passive marker device 10a and the second passive marker device 10b comprise a circuit element. Although Fig. 4 may show that the first passive marker device 10a and the second passive marker device 10b both comprise the circuit element 18d shown in Fig. 2D, it will be appreciated that the first passive marker device 10a and the second passive marker device 10b may comprise other circuit element, e.g., the circuit element 18a-18c, and 18e-18g shown in Fig. 2 to limit a power. In some examples, as shown in FIG. 4, the first passive marker device 10a and the second passive marker device 10b may comprise the same circuit element. In some other examples (not shown), the first passive marker device 10a and the second passive marker device 10b may comprise different circuit elements. For example, the first passive marker device 10a may comprise the circuit element 18a shown in Fig. 2D, while the second passive marker device 10b may comprise the circuit element 18f shown in Fig. 2F.

In some examples, the first passive marker device 10a and the second passive marker device 10b shown in Figs. 3A and 4 may differ by operating frequencies. For example, the first mechanical resonator 14a of the first passive marker device 10a may have a mechanical resonance frequency of *f*₁, and the second mechanical resonator 14b of the second passive marker device 10b may have a mechanical resonance frequency *f*₂. The frequency spacing, i.e., |*f*₁-*f*₂|, may be equal to or greater than a threshold value. It is thus possible to use frequency selective excitation pulses to operate the first passive marker device 10a and the second passive marker device 10b at different frequencies.

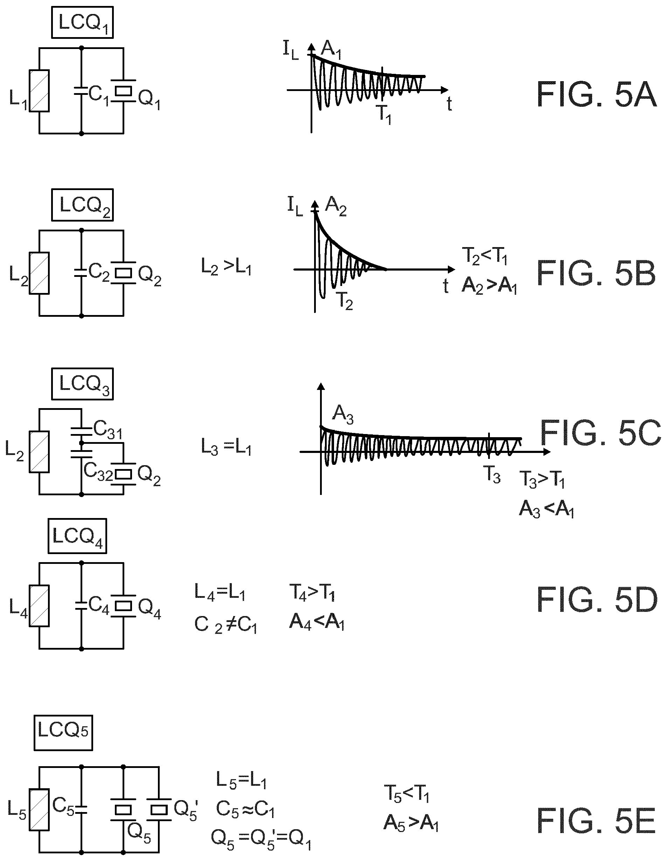

In some examples, the first passive marker device 10a and the second passive marker device 10b shown in Figs. 3A and 4 may have essentially the same operating frequency and different decay time constants. For example, the first mechanical resonator 14a of the first passive marker device 10a may have a mechanical resonance frequency *f*₁, and the second mechanical resonator 14b of the second passive marker device 10b may have a mechanical resonance frequency *f*₂. The frequency spacing, i.e., |*f*₁-*f*₂|, is less than a threshold value. For example, the first passive marker device 10a may have a first decay time constant *τ₁*, and the second passive marker device may have a second decay time constant *τ₂*. The difference between the two decay time constants, i.e., | *τ₁- τ₂*|, may be equal to or greater than a defined threshold value. In this way, the decay behaviors of the oscillation of the first passive marker device 10a and the second passive marker device 10b may be used as a distinguishing factor. Thus, it is possible to distinguish between two passive marker devices in the passive marker arrangement, even when they operate at the same frequency e.g., by fitting of the known response to the recorded data. The decay constant can be tuned by a suitable matching of the LC resonator with the mechanical resonator. In this matching, the product of inductance with capacitance is more or less fixed and is given by the mechanical resonance frequency. However, the ratio L/C is a design parameter that can be chosen freely. When the LC parallel resonator has a high resistance (in resonance), the time constant of the decay is low, however, the initial send amplitude is high. Overall, the total SNR is not changed, if the quality factor of the coil element is kept constant, which means the physical size of the coil element is unaltered. Figs. 5A-5E show exemplary circuits to change the time constant of the signal decay after excitation. The exemplary circuitry shown in Fig. 5A may be implemented as the first passive marker device 10a, for example. The passive marker device 10b may comprise one of the circuits shown in Figs. 5B to 5E to achieve a different time constant of the signal decay after excitation.

Fig. 5A shows the base circuitry to which the following circuits shall be compared. As shown in Fig. 5A, the base circuitry is also referred to as LCQ₁. The exemplary passive marker device shown in Fig. 5A comprises a coil element of inductance *L₁,* a capacitive element of capacitance Ci, and a mechanical resonator, such as a quartz resonator with a quality factor *Q.* In this example, the LC resonator formed by the inductor, the capacitor and the stray capacitances of inductor and quartz may be tuned to the same frequency as the natural frequency of the quartz. In all subsequent circuits, the mechanical resonator is keep the same i.e., Q₁=Q₂=Q₃=Q₄=Q₅. After a fixed excitation period e.g., with same frequency, length, and amplitude, the signal drops (in first approximation) with a characteristic decay time constant τ₁.

Fig. 5B shows the situation when the inductivity is replaced by a larger one, i.e., *L₂*>*L₁* while maintaining the quality factor of the coil - that is, the ratio of inductance to series resistance at the given frequency is kept constant. The exemplary passive marker device shown in Fig. 5B is also referred to as LCQ₂. Usually, this means that *L₁* and L2 can have roughly the same physical dimensions. The increase in inductivity increases the impedance of the tank circuit formed by *L* and *C* and therefore couples the quartz stronger to the tank circuit. The result is a shortened time constant, i.e., τ₂<τ₁, and increased amplitude at the beginning i.e., A2>A1. Together, the total SNR does not change much. So, by adjusting the inductance, the shape of the response can be altered. The shape of the response is inherent to the passive marker device and can be known beforehand or be measured during the acquisition.

Fig. 5C shows a circuit to reduce the coupling between the tank circuit and the quartz by a capacitive voltage divider. The exemplary passive marker device shown in Fig. 5C is also referred to as LCQs.In this example, the inductivity remains unchanged, i.e., *L₃*=*L₁,* while maintaining the quality factor of the coil. The capacitive voltage divider formed by two capacitors C₃₁ and C₃₂ reduces the coupling between the tank circuit and the quartz. The result is an extended time constant, i.e., *τ₃*>*τ₁*, and decreased amplitude at the beginning, i.e., A3<A1.

Fig. 5D shows an exemplary circuit to decrease the coupling between quartz and tank circuit by a slight detuning of the tank circuit. The exemplary passive marker device shown in Fig. 5D is also referred to as LCQ₄. This means the LC resonator formed by the inductor, the capacitor and the stray capacitances of inductor and quartz is not tuned to the same frequency as the natural frequency of the quartz. In this example, the capacitance *C*₄ is different from the capacitance *C*₁ shown in Fig. 1, while maintaining the same inductance, i.e., *L*₄=*L*₁*.* As the LC resonator is not tuned to the same frequency as the natural frequency of the quartz, the signal drops (in first approximation) with an elongated decay time constant τ₄, i.e., τ₄>τ₁, and decreased amplitude at the beginning, i.e., A₄<A₁.

Fig. 5E shows a further exemplary circuit to achieve a shorter τ by using two or more quartz crystals in parallel. The exemplary passive marker device shown in Fig. 5E is also referred to as LCQs.Although Fig. 5E may show two quartz crystals, namely Q₅ and Q₅', by way of example, it will appreciated that it is possible to parallel three, four, or more quartz crystals for some other implementations. Such configuration may be beneficial to increase the inductance of the coil indefinitely. Indeed, the inductance may be limited by manufacturing issues like unpractically thin conductors and the self-resonance of the coil. An alternative to paralleling quartz crystals is to use a larger quartz crystal (not shown).

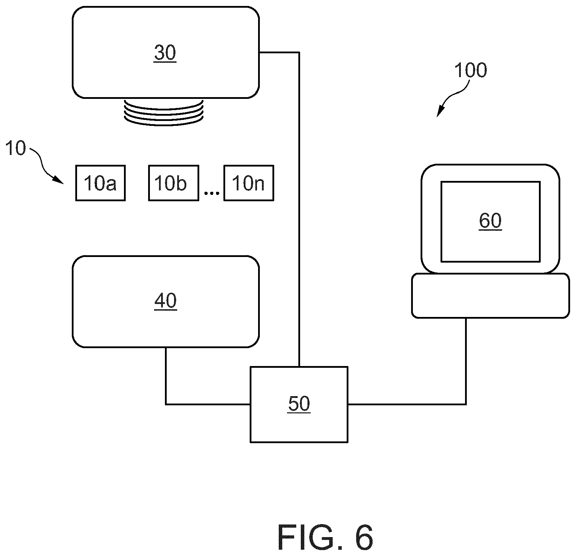

Fig. 6 schematically shows an example of a tracking system 100. The tracking system 100 comprises a plurality of passive marker devices 10, an excitation field generator 30, and a tracking device 40. In some examples, as shown in Fig. 6, the tracking system 100 may further comprise a position determination device 50,

As shown in Fig. 6, the plurality of passive marker devices 10 may comprise passive marker devices 10a, 10b,..., 10n, where n is equal to or greater than 2. In some examples, the plurality of passive marker devices 10, such as passive marker devices 10a, 10b,..., 10n shown in Fig. 6, may comprise one or more exemplary passive marker devices shown in Fig. 1. In some examples, In some examples, the plurality of passive marker devices 10, such as passive marker devices 10a, 10b, ..., 10n shown in Fig. 6, may comprise one or more exemplary passive marker devices shown in Figs. 2A-2H. In some examples, the plurality of passive marker devices 10, such as passive marker devices 10a, 10b,..., 10n shown in Fig. 6, may comprise one or more exemplary passive marker device arrangement shown in Figs. 3A and 4. For example, two of the plurality of plurality of passive marker devices may be mounted on a rigid mechanical arrangement, e.g., a housing, to track 6 DOF of movement of a device, e.g., a medical device, in three-dimensional space. In some examples, the plurality of passive marker devices 10 may comprise any combination of exemplary passive marker devices shown in Fig. 1, Figs. 2A-2H, Fig. 3A, and Fig. 4. In some examples, the plurality of passive marker devices 10a, 10b,..., 10n may be attached to one or more devices (not shown), e.g., one or more medical devices, in order to track the position of the plurality of devices relative to the excitation field generator 30 and the tracking device 40.

The excitation field generator 30 is configured to generate at least one excitation pulse to excite at least two passive marker devices in simultaneous manner or in a sequential manner within one excitation time slot.

The tracking device 40 is configured to detect the response signals generated by the at least two passive marker devices and to differentiate the at least two passive marker devices on the basis of the operating frequencies of the response signals and the decay time constants of the response signals. The excitation and recording scheme will be discussed hereinafter and in particular with respect to the examples shown in Figs. 9 and 10.

In some examples, as shown in Fig. 6, the tracking system 100 may further comprise a position determination device 50 configured to receive the response signals from the tracking device 40 and to receive position information from the excitation field generator 30. Based on the received response signals and the position information from the excitation filed generator 30, the position determination device 50 can determine the position of the plurality of passive marker devices 10 and, hence, the position of the device(s) to which the plurality of passive marker devices are attached, relative to the excitation field generator 30 and the tracking device 40. In some examples, the position determination device 40 may implement a gradient-based tracking approach employing saturation of the coil elements in the passive marker devices. For example, the excitation field generator 30 may generate an external saturating field, which causes the coil elements to be saturated. Hereby, this saturating field is provided with a gradient. This gradient means that the amplitude picked up by the tracking device 40 will be different depending on the position and orientation at which the passive marker device 10 is provided in the gradient field. Accordingly, the measured amplitude may allow to restrict the position of the passive marker device to a certain area, such as a certain plane, in the excitation field by correlating the position information provided from the excitation field generator 30 and the tracking device 40.

In some examples, as shown in Fig. 6, the tracking system 3 may further comprise a display configured to generate e.g., a graphical representation of the tracking and output this graphical representation to a user. Using this arrangement, it is possible to provide a tracking system having a high frequency resolution and a high quality factor for both, small and large dimensions, i.e. independent of the dimensioning of the devices.

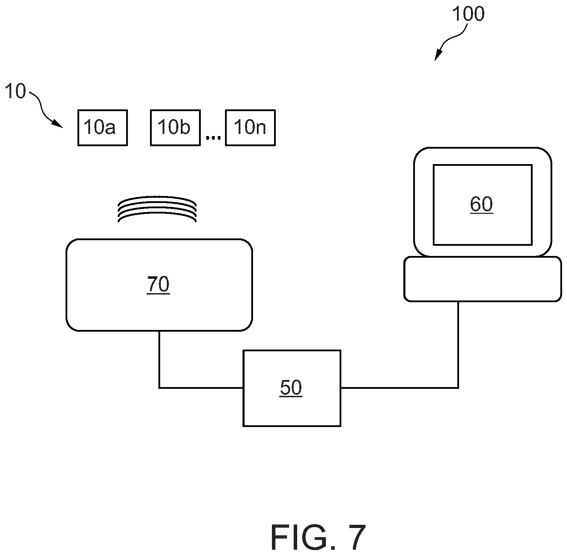

Fig. 7 schematically and exemplary shows a further example of a tracking system 100. In general, the exemplary tracking system 100 according to Fig. 7 works in the same fashion as the embodiment according to Fig. 6. Insofar it shall be referred to Fig. 6 for sake of brevity. The difference between the embodiment of Fig. 7 and the embodiment of Fig. 6 as discussed above, resides in the fact that, in the example according to Fig. 7, the excitation and field generator 30 and the tracking array 40 are provided as a single magnetic array 70. That is, in the example according to Fig. 7, the same magnetic array that is used to generate the excitation field and/or the saturating field is also used for picking up the oscillation response by the plurality of passive marker devices 10, e.g., passive marker devices, 10a, 10b,... ,10n as shown in Fig. 7. For this purpose, the magnetic array 70 may comprise a circuit that allows to switch between a transmission mode, in which the excitation field and/or the saturating field is generated and provided to act on the plurality of passive marker devices 10 and a reception mode, in which the magnetic field generated by the plurality of passive marker devices 10 may be picked up. As stated, the position determination device and the display 60 are the same as described in relation to Fig. 6, only that there is no communication between two separate units, i.e. the excitation field generator 30 and the tracking device 40 necessary, as the magnetic array 70 already is aware of the values and positioning of the excitation field and/or the saturating field generated.

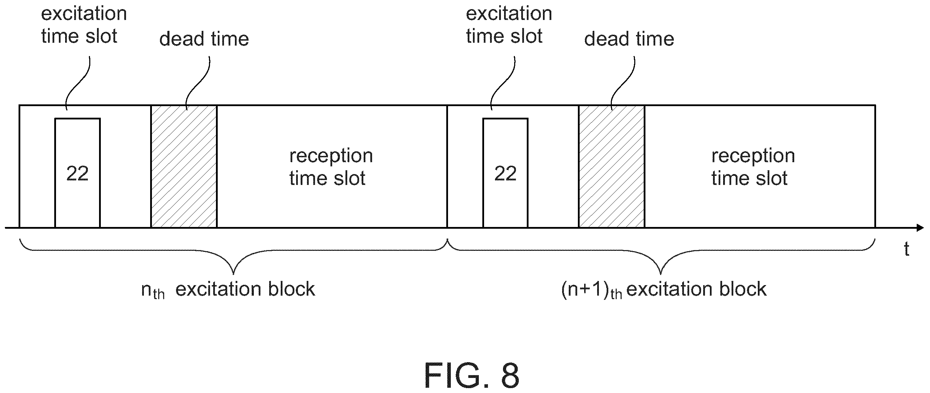

Fig. 8 illustrates a general excitation and recording scheme. As shown in Fig. 8, a plurality of excitation pulses 22 form an excitation sequence. Although Fig. 8 may show two excitation pulses 22 by way of example, it will be appreciated that the excitation sequence may comprise more excitation pulses 22. As it is technically challenging, excitation and reception is usually not done simultaneously. Rather, as shown in Fig. 8, there may be a dedicated time for sending, and thereafter for reception. In the following, the dedicated time for sensing is also referred to as excitation time slot or send phase, while the dedicated time for reception is also referred to as reception time slot or receive phase. As shown in Fig. 8, there may be an unavoidable dead-time between the excitation time slot and the reception time slot. Due to this dead time, it may be not feasible to switch very rapidly between sending and receiving. The excitation time slot, the dead time, and the reception time slot form an excitation block. In the example shown in Fig. 8, two consecutive excitation blocks, e.g., nₜₕ excitation block and (n+1)ₜₕ, excitation block, are illustrated. The dead time may be a major limitation and may prevent the use of other excitation schemes. It should also be noted that, at the end, there might an additional dead time between the reception time slot and the following excitation time slot. However, it is possible to construct electronic circuits to reduce this second dead-time time. The switching time is in the order of roughly one millisecond. For some simple electronics it may be longer. With precisely tuned, and hence advanced electronics, 100µs may be achievable.

Fig. 9 illustrates the basic principle of an exemplary improved excitation scheme. In this example, the improved excitation scheme will be explained with respect to two passive marker devices 10a and 10b. The two passive marker devices 10a and 10b may differ by the operating frequencies and/or decay time constants. For example, the mechanical resonance frequency of the mechanical resonator of the passive marker device 10a is *f*₁, and the mechanical resonance frequency of the mechanical resonator of the passive marker device 10b is *f*₂. The time constant of the passive marker device 10a may be larger than the time constant of the passive marker device 10b, i.e., *τ*₁>*τ*₂. For example, the difference between the time constants of the two passive marker devices 10a and 10b may be equal to or greater than a defined threshold value. As there is a dead time, it may be most efficient to have only one excitation phase, acting jointly on both passive marker devices, and then measure both markers during one reception phase. For example, as shown in Fig. 8, two frequency selective excitation pulses 22a and 22b may be generated to excite the two passive marker devices 10a and 10b in a sequential manner. The frequency component of the frequency selective excitation pulse 22a may be *f*₁ or close to *f*₁ to excite the mechanical oscillations of the mechanical resonator of the passive marker device 10a in the resonant mode. The frequency component of the frequency selective excitation pulse 22b may be *f*₂ or close to *f*₂ to excite the mechanical oscillations of the mechanical resonator of the passive marker device 10b in the resonant mode. Both frequency selective excitation pulses 22a and 22b are generated within one excitation time slot to excite the two passive marker devices 10a and 10b in a sequential manner. In addition, it may be efficient to sort the excitation in a way that the longer lasting passive marker device is excited first, then the less long-lasting passive maker device. This may ensure a better over-all SNR. In the example shown in Fig. 8, the passive maker device 10a is a longer lasting passive maker device because the time constant of the passive marker device 10a may be larger than the time constant of the passive marker device 10b, i.e., *τ*₁>*τ*₂. For this reason, the passive marker device 10a may be excited first by the frequency selective excitation pulse 22a, and then the passive marker device 10b, which is excited by the frequency selective excitation pulse 22b. As the passive marker device 10b is excited later, the response signal generated by the passive marker device 10b has less decay in the reception time slot, thereby improving SNR.

Fig. 10 illustrates the basic principle of another exemplary improved excitation scheme. In general, the exemplary improved excitation scheme according to Fig. 10 works in the same fashion as the embodiment according to Fig. 9. The difference between the embodiment of FIG. 10 and the embodiment of Fig. 9 as discussed above, resides in the fact that the mechanical resonance frequency of the mechanical resonator of the passive marker device 10a is *f*₁, and the mechanical resonance frequency of the mechanical resonator of the passive marker device 10b is also *f*₁ or close to *f*₁. The two passive marker devices 10a and 10b differ by decay time constants. In other words, this example uses two passive marker devices 10a and 10b at the same frequency but with different decay times such that at least one of the passive marker device, e.g., passive marker device 10b shown in Fig. 10, has significantly decayed before re-excitation. In this case, both passive marker devices 10a and 10b can be excited efficiently with the same excitation pulse 22 and the phase of the excitation may need (if at all) only to match with the longer time-constant resonator, e.g., passive maker device 10a shown in Fig. 10. This can achieve a very efficient mode of excitation. This exemplary excitation scheme can be combined with frequency selective excitation to result in a very efficient excitation scheme.

In some examples, the two passive maker devices 10a and 10b shown in Figs. 9 and 10 may be arranged in a similar manner as the exemplary passive marker device arrangement show in Figs. 3A and 4 and attached to a single device.

Although Figs. 9 and 10 illustrate the basic principle of exemplary improved excitation scheme for two passive marker devices by way of example, it will be appreciated that the excitation scheme may also be applied to track three or more passive marker devices. However, if there are more than two passive marker devices present, it may be necessary not to excite them in one joint excitation phase (i.e., in one excitation time slot) as too much decay may already occur during the excitation phase for some passive marker devices. Therefore, it may be more beneficial to combine only some of them into excitation pulses and then go through them in the default "round robin" way. If the frequencies are close to each other, the first excitation pulse used to excite the first passive marker device will also influence the second passive marker device. This may be considered and used as an advantage by timing the excitations in a way to constructively interfere. This may possible if the second excitation pulse starts on a very low level of oscillation amplitude, i.e., when its corresponding τ is low compared to the repetition time of the full sequence of excitations. Then, the phase of the excitation becomes a free parameter and phase matching is easily performed. This may be a special case when two or more passive marker devices in one excitation block operate at the same frequency. In such case, it may be desired to have the repetition time so long that all except one passive marker devices have short τ constants so that only the phase of the one passive marker device is not free. This passive marker device may be excited in the right phase and all other are automatically excited in the right phase as for them the phase is not relevant.

Up to now, it was implicitly assumed that all passive marker devices are aligned in a way that they can be excited with the same coil array settings, e.g., fixed amplitude and phase correlation in all excitation coils. However, for a tracking system there are several passive marker devices present in such a way that the excitation vector at the position of passive marker devices may be generated in all spatial directions. Therefore, there are more freedoms in sequence generation. Therefore, it is for example possible to excite two passive marker devices simultaneously, if the coil current pattern is distinct enough. However, this cannot be relied upon in most cases. Nevertheless, in some instances it is possible to rely on this when the orientation of the two passive marker devices are physically linked. One example for this is a 6 DOF sensor. Two examples of the 6 DOF sensor are shown in Figs. 3A and 4. Insofar it shall be referred to Figs. 3A and 4 for sake of brevity. Here, two passive marker devices are arranged in a way that their magnetization vectors are non-parallel to each other, preferably orthogonal or almost orthogonal (e.g., in a range from about 85° to about 95°) to each other. This arrangement, such as the arrangement shown in Figs. 3A and 4, could be excited at the same time in one excitation time slot and the two orientations could have different frequencies if different frequencies are necessary in the application.

As noted above, one problem is the possibility of too high signal amplitudes of a passive marker device close to the excitation field generator. This may be due to the operation of two or more passive marker devices at the same frequency and one being much closer to the coil array than the others. This may also happen due to the operation of two non-parallel passive maker devices, e.g., the passive marker device arrangement shown in Figs. 3A and 4, and one being parallel to the local excitation field and the other being orthogonal or almost orthogonal (e.g., in a range from about 85° to about 95°) to the local excitation field. It may also happen if they are not tuned to the same frequency but subject to spectral leaking. This must be resolved by hardware modifications of the passive marker devices and an adaptation of the sequence.

Examples of the hardware modifications of the passive marker devices 10 are shown in Figs. 2A-2H. Insofar it shall be referred to Figs. 2A-2H for sake of brevity.

As discussed with respect to Figs. 2D to 2H, it is possible reduce or even eliminate the coupling between the coil element and the mechanical resonator for a duration much longer than the oscillation period. The reason to change the coupling between the coil element and mechanical resonator are twofold: first not to destroy the mechanical resonator, and second not to saturate the receive amplifier (or have excessive spectral leakage). For the latter reason, a suitable sequence has to be provided. Assuming a simple circuit where the amplitude is capped, the sequence during the send phase (i.e., in the excitation time slot) has two phases: first a low amplitude signal is sent, then a higher amplitude signal having about the same duration, but with opposite phase is sent. This has the effect, that at the near position, due to capping of the signal, a near zero excitation is achieved. At the position far away from the coil array, the relative amplitudes of the two 180° phase-shifted signal do not cancel and hence a relatively high signal amplitude is achieved. This is because at this far-away position the excitation is linear with the applied field and the weak field from the first phase does not cancel the strong field from the second phase. The exact time ratio of the low and high amplitude signal may depend on the residual level of the wanted excitation and the achieved residual amplitudes at the mechanical resonators during the phases with different amplitudes. In these circuits, the amplitude at the mechanical resonator may still rise a little, when the excitation amplitude is increased, even when being in the clipping mode. Therefore, in these circuits, a relatively long time may be consumed during ineffective excitation. The transistor (or varactor) based circuits may offer a better efficiency as they can reduce the effective excitation level much further, especially, if a frequency component off resonant to the mechanical resonating frequency keeps them in a deeply switched-off state.

Now a set of excitations is present that would be sufficient if the read-out phase would begin immediately. However, for a high number of passive marker devices, the excitation phase may be so long that some of the signal has decayed too much before the reception phase (i.e., the reception time slot). The first step to avoid this may be to sort the excitations according to decay time with the shortest decay time coming latest. If this is not sufficient, the excitation phase must be split into several. During sorting and splitting simultaneous excitations are kept as a single unit. When splitting the sequence, constituents are picked from the source sequence in order and then put in the next sub-sequence. So, for example if the initial pulse sequence goes like "22a, 22b, 22c, 22d, 22e, 22f, 22g, 22h, 22i, 22j, 22k", the distribution in two sequences would be "22a, 22c, 22e, 22g, 22i, 22k" and "22b, 22d, 22f, 22h, 22j". Distribution in three would result in "22a, 22d, 22g, 22j", "22b, 22e, 22h, 22k", and "22c, 22f, 22i". The 180°phase flip excitations are kept within the same excitation window, so this splitting rule has to be adapted accordingly.

Note that the computations need to consider the initial state of the mechanical resonator. Especially for the long decay time passive marker devices the excitation builds up gradually. An additional consideration may be that there are initially build-up processes and for the beginning, it may not be possible to meet wanted signal levels or there is a need to split up in many more excitation windows. The software must allow this before concluding that excitation cannot fulfill the desired quality standard. The excitation should be aligned in a way to constructively add more energy to the quartz. The final excitation level may not be exactly the desired one, but in most application a few 10% deviation in the amplitude and phase of the signal does not hurt. The structure of the sequence may not need to be computed every time for most applications. In the applications the passive marker devices may not move very fast and hence the structure remains constant. Only the specific amplitudes and durations may be varied. This also allows for the computation of the sequence structure to take much more time than the repetition time and hence less powerful computers may be used.

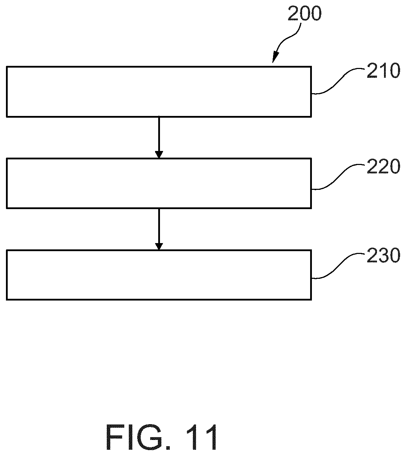

Fig. 11 illustrates a flowchart describing a method for tracking a plurality of marker device. The method steps will be described in connection with the tracking system shown in Figs. 6 and 7.

In step 210, the method comprises generating at least one excitation pulse to excite at least two passive marker devices in simultaneous manner or in a sequential manner within one excitation time slot. The at least one excitation pulse may be generated by the excitation field generator 30 shown in Fig. 6 or by the magnetic array 70 shown in Fig. 7.

In some examples, the plurality of passive marker devices may comprise two or more passive marker devices having operating frequencies with a frequency spacing equal to or greater than a threshold value. The excitation field generator may be configured to generate a sequence of frequency selective excitation pulses within the excitation time slot to excite at least two of the two or more passive marker devices in a sequential manner. For example, Fig. 9 schematically shows an exemplary excitation scheme to generate two frequency selective excitation pulses 22a and 22b within the excitation time slot to excite two passive maker devices 10a and 10b in a sequential manner. The excitation field generator may sort the frequency selective excitation pulses according to the decay time constants of the at least two passive marker devices to be excited. For example, in the example shown in Fig. 9, the passive maker device 10a is a longer lasting passive maker device because the time constant of the passive marker device 10a may be larger than the time constant of the passive marker device 10b, i.e., *τ*₁>*τ*₂. For this reason, the passive marker device 10a may be excited first by the frequency selective excitation pulse 22a, and then the passive marker device 10b, which is excited by the frequency selective excitation pulse 22b. As the passive marker device 10b is excited later, the response signal generated by the passive marker device 10b has less decay in the reception time slot, thereby improving the SNR. Insofar it shall be referred to Fig. 9 for sake of brevity.

In some examples, the plurality of passive marker devices may comprise two or more passive marker devices configured to have operating frequencies with a frequency spacing less than a threshold value and to have different decay time constants. For example, the exemplary excitation scheme shown in Fig. 10 uses two passive marker devices 10a and 10b at the same frequency but with different decay times such that at least one of the passive marker device, e.g., passive marker device 10b shown in Fig. 10, has significantly decayed before re-excitation. In this case, both passive marker devices 10a and 10b can be excited efficiently with the same excitation pulse 22 and the phase of the excitation may need (if at all) only to match with the longer time-constant resonator, e.g., passive maker device 10a shown in Fig. 10. Insofar it shall be referred to Fig. 10 for sake of brevity.

In addition, as described above, if there are more than two passive marker devices present, it may be necessary not to excite them in one joint excitation phase (i.e., in one excitation time slot) as too much decay may already occur during the excitation phase for some passive marker devices. Therefore, it may be more beneficial to combine only some of them into excitation pulses and then go through them in the default "round robin" way.

In step 220, the method comprises detecting one or more response signals generated by the plurality of passive marker devices. The one or more response signals may be detected by the tracking device 40 shown in Fig. 6 or by the magnetic coil 70 shown in Fig. 7. The plurality of passive marker devices may be differentiated on the basis of the operating frequencies of the response signals and the decay time constants of the response signals.

In some examples, as described with respect to the exemplary excitation and recording schemes shown in Figs. 9 and 10, the response signals may be detected in one reception time slot.

In some other examples, for a high number of passive marker devices, the excitation phase may be so long that some of the signal has decayed too much before the reception phase (i.e., the reception time slot). In such cases, the response signals generated by the two or more passive marker devices may be detected in a plurality of reception time slots according to the decay time constants of the response signals.

In step 230, the method comprises determining the position of the plurality of passive marker devices based on the one or more response signals. The position may be determined by the position determination device 40 shown in Figs. 6 and 7. In some examples, the position determination device 40 may implement a gradient-based tracking approach employing saturation of the coil elements in the passive marker devices. For example, the excitation field generator 30 may generate an external saturating field, which causes the coil elements to be saturated. Accordingly, this saturating field is provided with a gradient. This gradient means that the amplitude picked up by the tracking device 40 will be different depending on the position and orientation at which the passive marker device 10 is provided in the gradient field. Accordingly, the measured amplitude may allow to restrict the position of the passive marker device to a certain area, such as a certain plane, in the excitation field by correlating the position information provided from the excitation field generator 30 and the tracking device 40.

In another exemplary embodiment of the present invention, a computer program or a computer program element is provided that is characterized by being adapted to execute the method steps of the method according to one of the preceding embodiments, on an appropriate system. The computer program or computer program element may include instructions that are executable by a computer unit.

The computer program element might therefore be stored on a computer unit, such as a computer, which might also be part of an embodiment of the present invention. For example, the computer program element may be stored in a storage unit such as a memory. Such computing unit may be adapted to perform or induce a performing of the steps of the method described above. Moreover, it may be adapted to operate the components of the above described apparatus. The computing unit can be adapted to operate automatically and/or to execute the orders of a user. In some embodiments, the computing unit includes one or more processors. The one or more processors may be a data processor. A computer program may be loaded into a working memory of a data processor or processing circuit. The data processor may thus be equipped to carry out the method of the invention.

This exemplary embodiment of the invention covers both, a computer program that initially uses the invention and a computer program that by an up-date turns an existing program into a program that uses the invention.

Further on, the computer program element might be able to provide some or all necessary steps to fulfil the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section. In some embodiments, the computer readable medium is non-transitory.

A computer program may be stored and/or distributed on a suitable medium, such as an optical storage medium or a solid state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfil the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. A passive marker device (10), comprising:
- a coil element (12);
- a mechanical resonator (14); and
- a circuit element (16);
wherein the coil element is coupled to the mechanical resonator for producing a response signal having an operating frequency corresponding a mechanical resonating frequency of the mechanical resonator and a signal decay having a decay time constant in response to an excitation pulse, wherein the excitation pulse is a magnetic or electromagnetic excitation pulse; and
wherein the circuit element is configured to limit a power transferred to the mechanical resonator when the at least one excitation pulse has an excitation amplitude equal to or greater than a threshold value.

2. The passive marker device according to claim 1,
wherein the circuit element comprises at least one of:
- a diode configured to limit a voltage across the mechanical resonator;
- a plurality of diodes connected in series configured to limit a voltage of the mechanical resonator; or
- a pin diode configured to limit a voltage across the mechanical resonator.

3. The passive marker device according to claim 1,
wherein the circuit element is configured to reduce a coupling between the coil element and the mechanical resonator.

4. The passive marker device according to claim 3,
wherein the circuit element comprises a varactor diode and a resistor parallel to the varactor diode.

5. The passive marker device according to claim 4,
wherein the circuit element further comprises one of:
- a field effect transistor configured to clamp a charging action;
- a field effect transistor configured to clamp a charging action and a series capacitor connected to the field effect transistor;
- a field effect transistor configured to clamp a charging action and a series capacitor connected to the field effect transistor, wherein the field effect transistor is connected to a capacitive voltage divider of the capacitive element; or
- a normally-on field effect transistor as a switching element.

6. A passive marker arrangement (20), comprising:
- a first passive marker device (10'); and
- a second passive marker device (10");
wherein each of the first and second passive marker device comprises a coil element and a mechanical resonator, wherein the coil element is coupled to the mechanical resonator for producing a response signal having an operating frequency corresponding a mechanical resonating frequency of the mechanical resonator and a signal decay having a decay time constant in response to an excitation pulse, wherein the excitation pulse is a magnetic or electromagnetic excitation pulse; and
wherein the first passive marker device and the second passive marker device are mounted on a mechanical arrangement at an angle with respect to each other, such that the coil element of the first passive marker device is non-parallel to the coil element of the second passive marker device.

7. The passive marker arrangement according to claim 6,
wherein at least one of the first passive marker device and the second passive marker device is a passive marker device according to any one of claims 1 to 5.

8. The passive marker arrangement according to claim 6 or 7,
wherein the angle is in a range from about 45° to about 135°, optionally in a range from about 85° to about 95°, preferably about 90°.

9. The passive marker arrangement according to any one of claims 6 to 8,
wherein the first passive marker device and the second passive marker device are configured to have operating frequencies with a frequency spacing less than a threshold value and to have different decay time constants, or to have operating frequencies with a frequency spacing equal to or greater than a threshold value.

10. A tracking system (100), comprising:
- a plurality of passive marker devices (10a, 10b, ..., 10n), wherein the plurality of passive marker devices comprises a passive marker device according to any one of claims 1 to 5 and/or a passive marker arrangement according to any one of claims 6 to 9;
- an excitation field generator (30) configured to generate at least one excitation pulse to excite at least two passive marker devices in simultaneous manner or in a sequential manner within one excitation time slot; and
- a tracking device (40) configured to detect the response signals generated by the at least two passive marker devices and to differentiate the at least two passive marker devices on the basis of the operating frequencies of the response signals and the decay time constants of the response signals.

11. The tracking system according to claim 10,
wherein the tracking device is configured to detect the response signals generated by the two or more passive marker devices in one or more reception time slots according to the decay time constants of the response signals.

12. The tracking system according to claim 10 or 11,
wherein the plurality of passive marker devices comprises two or more passive marker devices configured to have operating frequencies with a frequency spacing less than a threshold value and to have different decay time constants.

13. The tracking system according to any one of claims 10 to 12,
wherein the plurality of passive marker devices comprises two or more passive marker devices configured to have operating frequencies with a frequency spacing equal to or greater than a threshold value; and
wherein the excitation field generator is configured to generate a sequence of frequency selective excitation pulses within the excitation time slot to excite at least two of the two or more passive marker devices in a sequential manner.

14. The tracking system according to claim 13,
wherein the excitation field generator is configured to sort the frequency selective excitation pulses according to the decay time constants of the at least two passive marker devices to be excited.

15. The tracking system according to any one of claims 9 to 14,
wherein the excitation field generator is configured to generate a sequence of phase-altering excitation pulses to excite at least two passive marker devices having different distances to the excitation field generator; and
wherein the sequence of phase-altering excitation pulses comprises a first signal with a low amplitude and a second signal having a higher amplitude, wherein the first signal and the second signal have opposite phases.
